# EUROPÄISCHE PATENTANMELDUNG

(11) **EP 4 176 832 A1**
(43) Veröffentlichungstag der Anmeldung: **10.05.2023**
(21) Anmeldenummer: 22206222.6
(22) Anmeldetag: 08.11.2022
(51) Int. Cl.: A61B 17/72

(54) **MARKNAGEL**

(30) Priorität: 08.11.2021 EP 21206917; 09.12.2021 EP 21213501
(71) Anmelder: Bechtold, Daniel, 72458 Albstadt-Ebingen (DE)
(72) Erfinder: Bechtold, Daniel, 72458 Albstadt-Ebingen (DE)
(74) Vertreter: Jeck, Jonathan

(57) **Zusammenfassung**

Die vorliegende Erfindung betrifft einen rotationsstabilen Marknagel für einen Röhrenknochen mit einer Längsachse, wobei dieser mindestens eine sich zumindest teilweise entlang der Längsachse erstreckende Kante aufweist und mehrteilig aufgebaut ist.

## Beschreibung

Die vorliegende Erfindung betrifft einen Marknagel gemäß Anspruch 1. Bevorzugte Ausführungsformen sind in den abhängigen Ansprüchen angegeben.

Bisherige Marknagelsysteme beruhen darauf, dass ein runder Nagel als Ganzes von außen bzw. vom Frakturspalt her in die Markhöhle eingedreht wird. Ausnahme: Trilamnagel, der eingeschlagen wird.

Solche Systeme haben den Nachteil, dass keine ausreichende Rotationsstabilität gegeben ist. Das heißt, die beiden Bruchfragmente können gegeneinander verdreht werden. Eine Ausnahme hierbei bilden Verriegelungsnägel bei denen von außen Schrauben durch den Knochen und den Marknagel getrieben werden.

Die vorliegende Erfindung stellt sich die **Aufgabe,** einen Marknagel bereit zu stellen, welcher die Aufrichtung eines gebrochenen Röhrenknochens mit gleichzeitiger Rotationsstabilisierung ermöglicht.

Diese Aufgabe wird durch einen Marknagel mit den Merkmalen des Anspruchs 1 gelöst.

Erfindungsgemäß ist ein Marknagel mit einer Längsachse für einen Röhrenknochen vorgesehen, wobei der Marknagel einen zweiteiligen länglichen Grundkörper aufweist, welcher sich im Wesentlichen über die Länge des Marknagels erstreckt.

Der erfindungsgemäße Marknagel ist dadurch gekennzeichnet, dass der Grundkörper mit einem sich entlang seiner Längsachse erstreckenden Kreuz- oder Sternprofil gebildet ist, welches im Wesentlichen sich entlang der Längsachse erstreckende Längskanten (3) aufweist, also der Grundkörper eine sich entlang seiner Längsrichtung erstreckende Außenkontur, mit einer Kreuz- oder Sternform im Querschnitt, aufweist, dessen Längskanten (3) sich parallel zur Längsrichtung des Grundkörpers erstrecken.

Bevorzugt ist es vorgesehen, dass die beiden Teile des Grundkörpers an einander zugewandten Endseiten jeweils einen Teil eines Verschlussmechanismus zum Verbinden der Grundkörperteile miteinander aufweisen und dass ein erster Teil des Verschlussmechanismus das Profil und/oder die Kontur des Grundkörpers, also die Kreuz- oder Sternform, aufweist und der zweite Teil des Verschlussmechanismus einen Aufnahmekörper (5) mit längs gerichteten Schlitzen aufweist, welche ihrer Form nach dem ersten Teil entsprechen.

Bevorzugte Ausführungsformen sind in den abhängigen Ansprüchen angegeben.

Nach der vorliegenden Erfindung handelt es sich um einen innovativen Marknagel (MKN) dessen Querschnitt vorzugsweise die Form eines Kreuz- oder Sternprofils aufweist. Der Querschnitt kann hierbei insbesondere als Schnitt quer zur Längsachse des Nagels, also zwischen dessen distalen und proximalen Ende verstanden werden. Dieser wird nun vom Frakturspalt aus in den Markraum (MKR) eingebracht. Dabei wird zunächst die MKR-Länge des oberen und unteren Röhrenknochenstückes gemessen. Dann wird der MKN auf die jeweiligen Längen gekürzt. Nun können am oberen und unteren Ende des MKN jeweils Platten die dem MKN-Durchmesser entsprechen aufgeschraubt werden. Diese sind auf der dem spongiösen Knochen zugewandten Seite mit, beispielsweise kreuzförmig angeordneten, Spitzen besetzt, welche beim Einschieben der beiden Marknägel nach oben bzw. nach unten in die knöchernen Enden des MKR eindringen und so den MKN rotationsstabil verankert. Nun kann im nächsten Schritt (nur bei Frakturen ohne Trümmer/ Splitter) eine Bohrführung auf jenen MKN gesetzt werden, welcher beiderseits als Kreuzprofil enden kann. Diese Bohrführung ermöglicht es jetzt Bohrlöcher exakt über den später darunter zu liegen kommenden Gewinden (im MKN) zu setzten. Der Durchmesser der Bohrlöcher entspricht dem Schraubendurchmesser. Es kann sich hierbei um schraubenkopflose Vollgewindeschrauben handeln, deren Länge dem MN-Durchmesser entsprechen. Vorzugsweise entspricht die Schraubenlänge dem MKN in dessen gewindetragenden Abschnitt. Vorzugsweise werden nun werden beide Marknägel(teile) ineinandergeschoben, wobei sich das (Kreuz)Profil des einen in ein entsprechendes Gegenstück (ein Negativ des (Kreuz)Profils) schiebt. Danach können die Schrauben von außen durch den nun geschlossenen und vollständig axial aufgerichteten Röhrenknochen in die Gewinde vorgetrieben und festgeschraubt werden. Der nun im MKR befindliche MKN wird durch die bestehende Muskelkontraktion regelrecht zwischen oberen und unteren Markraumende eingekeilt, wobei sich die mit Spitzen versehenen Enden des MKN tief im spongiösen Knochen verankern. Zusätzlich verstärkt wird dieser Effekt, bei der späteren Belastung der Gliedmaße. Vorgehsehen ist dieser MKN zunächst nur bei Frakturen des Oberschenkels vor allem bei Katzen (da deren Oberschenkelknochen im Vergleich zu jenen des Hundes weniger stark in deren Größe variieren). Eine spätere Anwendung bei Heimtieren (Nagern) und bei Mittelfuß- und Mittelhandknochenfrakturen bei Hunden und anderen Tieren ist ebenfalls denkbar. Weiterhin ist die Verwendung bei Menschen grundsätzlich möglich. Der Vorteil dieser Methode besteht in ihrer einfachen Anwendung im Vergleich zu alternativen chirurgischen Verfahren. Da hier lediglich der Weichteilmantel über der Fraktur eröffnet werden muss und keine weiteren Manipulationen am Gewebe vorgenommen werden müssen (Mobilisierung des Weichteilmantels zum Anbringen einer Platte). Dies führt zu einer deutlichen Verkürzung der OP-Zeit, was sich bekanntlich positiv auf den weiteren Heilungsverlauf (geringeres Infektionsrisiko) auswirkt. Dies ermöglicht es auch Tierärzten mit weniger fundierten chirurgischen Kenntnissen eine der häufigsten Frakturarten beim Kleintier zu versorgen und den Patienten nicht überweisen zu müssen. Ein weiterer Vorteil besteht darin, dass Patienten unmittelbar nach dem Eingriff die Gliedmaße wieder belasten können und somit lange Genesungsphasen wegfallen.

Es werden bei diesem System zwei Marknägel in der jeweiligen Länge des Bruchfragments vom Frakturspalt her in die Markhöhle eingebracht.

Vorzugsweise weist der Nagel 2 Teile auf. Diese Teile sind aneinander zumindest bezüglich einer Torsion zueinander oder im betriebsgemäßen Zustand, bezüglich des Marknagels um seine Längsachse, festlegbar. Vorzugsweise hat der Nagel beziehungsweise ein Teil des Nagels, zumindest bereichsweise im Querschnitt quer zur Längsrichtung des Nagels eine Kreuz- oder Dreiecksform. Eine andere Mehreckform oder Mehrspitzenform (3-X eckiger Stern) ist ebenfalls denkbar. Die beiden Teile des Marknagels sind in ihrer Form in Längsrichtung komplementär zueinander. Komplementär kann in diesem Zusammenhang insbesondere bedeuten, dass der erste Nagelteil im Verbindungsbereich eine Aufnahme aufweist, welche bezüglich seiner inneren Oberfläche der äußeren Form und Größenverhältnissen der Außenkontur des zweiten Marknagelteils, insbesondere in dessen Verbindungsbereich zum ersten Nagelteil, entspricht. Hierbei kann der eine Nagel in seiner äußeren Form das Negativ des anderen Nagels bilden.

Komplementär kann jedoch auch bedeuten, dass die beiden Marknagelteile zumindest in deren Verbindungsbereich 2 *Geometrien* aufweisen, welche ineinander (ver)fügbar sind. Hat der eine Teil des Nagels beispielsweise in seiner Querschnittsfläche eine Kreuzform (zumindest im Verbindungsbereich zum zweiten Teil des Nagels) kann der zweite Teil des Nagels eine beispielsweise 4-teilige Form in Längsrichtung aufweisen, wobei jeweils ein Teil der Form in einer Ecke des Kreuzes des anderen Teils des Marknagels aufliegt. Die 4-teilige Form kann 4 Vierecke oder 4 Dreiecke oder eine andere mehreckige Form aufweisen, welche mindestens einen rechten Winkel in Längsrichtung des Marknagels, bzw. den/die Winkel, welcher zwischen den einzelnen Flügeln der Kreuzform des ersten Teils des Nagels vorgesehen ist, enthalten kann.

Nach einer bevorzugten Ausführungsform können die beiden Nagelteile in ihrer längsform zueinander männlich-weiblich komplementär ausgebildet sein.

Insbesondere im Verbindungsbereich zwischen den beiden Marknagelteilen können Gewinde angeordnet sein, welche im betriebsgemäßen Zustand koaxial übereinander liegen, wodurch diese mit einer entsprechenden Schraube verbindbar sind.

An dem Ende des jeweiligen Nagelteils, welches dem Knochen zugewandt ist (distales/proximales Ende des Knochens) können ein oder mehrere Verankerungselemente vorgesehen sein. Dabei kann es sich um eine oder mehrere Spitzen oder andere Geometrien handeln, welche geeignet sind, in dem spongiösen Knochen Halt zu finden. Zur Verbesserung des Halses des Marknagelteils in dem Knochen mittels der Verankerungselemente kann das Innere des Knochens zuvor mit einem Stempel geebnet und gefestigt werden.

Mittels des Stempels oder eines weiteren Werkzeugs kann ebenfalls die spätere Eindringtiefe des Marknagelteils in das jeweilige Knochenfragment bestimmt werden.

Grundsätzlich kann der erfindungsgemäße Marknagel auch mehr als 2 Teile aufweisen. Bei einer solchen Konstruktion hätte der Marknagel dann 2 Endteile, welche jeweils ein Verbindungsbereich zu einem benachbarten Marknagelteil aufweisen und einen oder mehrere innenliegende Marknagelteile, welche jeweils endseitig 2 Aufnahmen für benachbarte Marknagelteile aufweisen.

Die Erfindung wird nun anhand von Ausführungsbeispielen näher erläutert. Es zeigen:

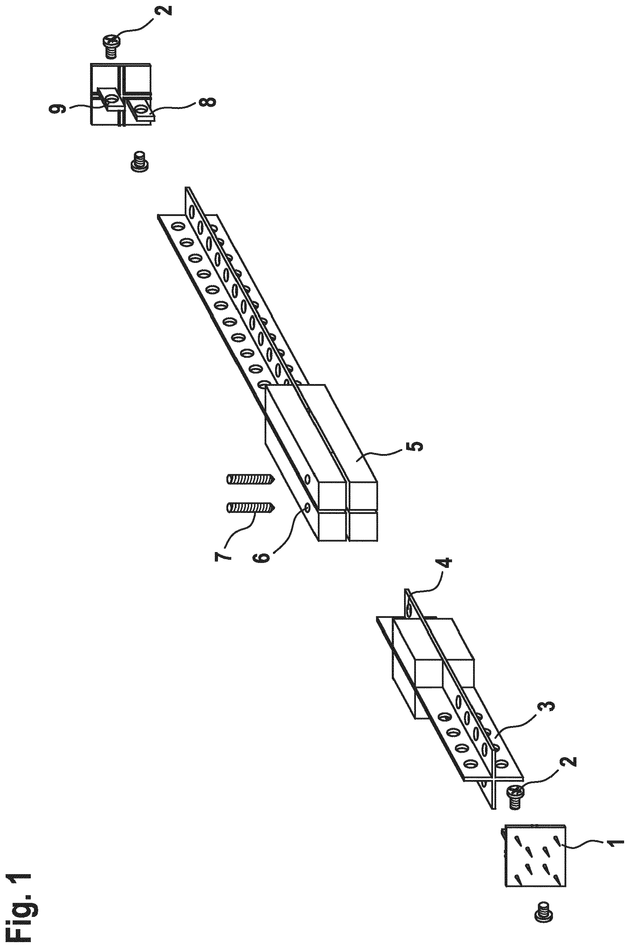

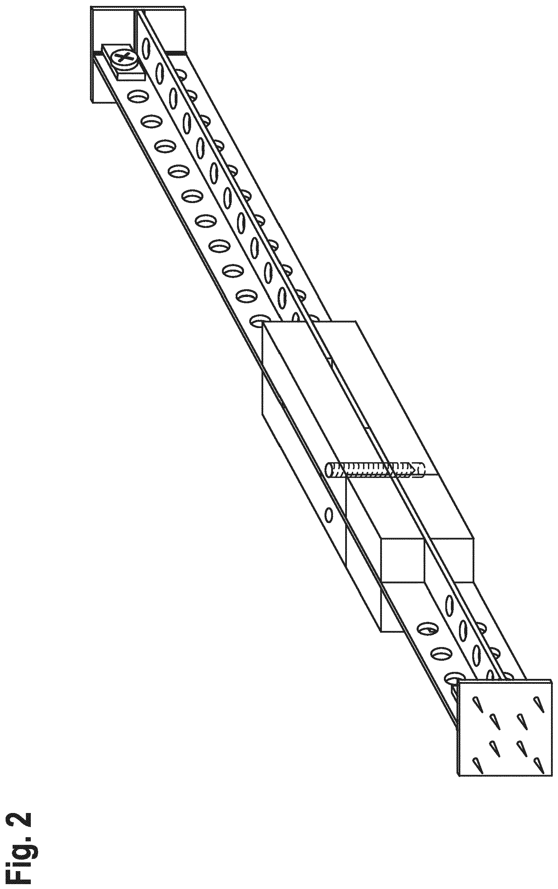

Die Figuren 1 bis 2 zeigen eine Ausführungsform des erfindungsgemäßen Marknagels.

Anstelle des voranstehend beschriebenen Kreuzprofils kann auch eine abweichende Geometrie des Marknagels verwendet werden, wie im allgemeinen Teil der Beschreibung diskutiert.

Bevorzugt ist es vorgesehen, dass die Länge des Grundkörpers an die Länge einer Markhöhle des Röhrenknochens anpassbar ist.

Bevorzugt ist es vorgesehen, dass der Marknagel zwei Teile mit jeweils einem Grundkörper aufweist, welche für jeweils ein Fragment eines Knochenbruchs vorgesehen sind.

Zweckmäßiger Weise kann es vorgesehen sein, dass die beiden Teile in ihrer Geometrie entlang der Längsachse komplementär zueinander ausgebildet sind. Besonders bevorzugt ist es vorgesehen, dass das eine Teil des Marknagels auf oder in das andere Teil stirnseitig formschlüssig schiebbar ist.

Nach einer zweckmäßigen Weiterbildung der Erfindung kann es vorgesehen sein, dass die Marknagelteile an ihrem distalen / proximalen Ende, welches bereitgestellt ist, in einen Knochen eingeführt zu werden, stirnseitig Verankerungselemente aufweist, welche ausgebildet sind, den Nagel im Knochen festzulegen.

Zweckmäßiger Weise kann es vorgesehen sein, dass die Marknagelteile an ihrem proximalen / distalen Ende stirnseitig mindestens ein abtrennbares Teil aufweisen.

Bevorzugter Weise kann es vorgesehen sein, dass der mindestens eine abtrennbare Teil einstückig mit dem jeweiligen Grundköper gebildet ist.

Besonders bevorzugt kann es vorgesehen sein, dass die Marknagelteile bei einem entfernten abtrennbaren Teil in ihrem proximalen / distalen Ende die zueinander komplementäre Geometrie aufweisen.

Vorzugsweise kann es vorgesehen sein, dass der Marknagelteil, welcher in den anderen Marknagelteil einführbar ist, an seinem Außenumfang einen Vorsprung aufweist, welcher eine maximale Eindringtiefe des einführbaren Marknagelteils in den anderen Marknagelteil definiert.

Zweckmäßiger Weise kann es vorgesehen sein, dass die beiden Marknagelteile miteinander verschraubbar sind.
Figur 1: Zeigt eine Explosionsansicht des Marknagels; und
Figur 2: Zeigt eine Ansicht des Marknagels in einem zusammengesetzten Zustand.

### Figur 1 Nr. 1

a.) Eine perspektivische Explosions- Vorderansicht der Endkappe mit Verankerungsspitzen.
b.) Eine perspektivische Explosions- Rückansicht der Endkappe mit kreuzförmiger Aufnahmefuge für das Kreuzprofil mit beidseitig paramedian liegenden Ösen.

### Figur 1 Nr. 2

Eine perspektivische Ansicht einer Schraube (mit Schraubenkopf) zur Fixierung der Endkappen am Kreuzprofil.

### Figur 1 Nr. 3

Eine perspektivische Explosionsansicht des Kreuzprofils mit (gewindetragenden) Bohrungen und einem mit dem Kreuzprofil bündig verbindbaren Anteil des Kreuzverschlusses, welcher dann nach dem männlich-/ weiblich-Prinzip in ein dem Kreuzprofil entsprechendes Gegenstück zur Aufnahme des selbigen eingeschoben werden kann.

### Figur 1 Nr. 4

Bohrung am Kreuzprofil im Bereich des Kreuzverschlusses zur Aufnahme von den beiden Vollgewindeschauben 7

### Figur 1 Nr. 5

Negativ des Kreuzprofils in welches das Kreuzprofil nach dem männlich-/ weiblich-Prinzip eingeschoben werden kann (Aufnahmekörper).

### Figur 1 Nr. 6

Eine Ansicht einer gewindetragenden Bohrung zur Aufnahme zweier Vollgewindeschrauben.

### Figur 1 Nr. 7

Ansicht einer Vollgewindeschraube (ohne Schraubenkopf) zur Fixierung des Kreuzverschlusses.

### Figur 1 Nr. 8

Öse zur Aufnahme einer schraubenkopftragenden Schraube zur Fixierung der Endplatten am Kreuzprofil.

### Figur 1 Nr. 9

### Kreuzförmige Fuge zur rotationsstabilen Fixierung des Kreuzprofils an der Endplatte

Zunächst werden die beiden Markhöhlenlängen des Knochens von der Fraktur her ausgemessen. Anschließend werden die Marknägel auf die entsprechenden Längen der Markhöhlen (vorzugsweise an ihren außenliegenden Enden) gekürzt. Die beiden Marknagelanteile werden von der Bruchstelle (des gebrochenen Knochens) her zur oberen und unteren Begrenzung der Markhöhle vorgeschoben. Dabei verankern sich die Längskanten 3 des Kreuzprofils des Marknagels an der inneren Knochenwandung der Markhöhle, wodurch dieser rotationsstabil festgelegt ist und (relative) Torsionskräfte zwischen den Knochenfragmenten aufnehmen kann. An der den Markhöhlenenden zugewandten Seite des Marknagels können jeweils eine mit Spitzen versehene Endkappe (Figur 1 Nr. 1) angebracht werden. Dabei kann das Marknagelende in eine dafür vorgesehene kreuzförmige Fuge auf der Rückseite der Endkappe (Figur 1 Nr. 9) zur Aufnahme des Kreuzprofils (Figur 1 Nr. 3) eingebracht werden. Anschließend kann die Endkappe 1 an mindestens einer, vorzugsweise zwei dafür vorgesehenen Ösen (Figur 1 Nr. 9), vorzugsweise mit jeweils einer Schraube (Figur 1 Nr. 2) beispielsweise über einer gewindetragenden Bohrung am Kreuzprofil fixiert werden. Nun werden die beiden Marknagelanteile (Figur 1 Nr. 3) jeweils in die entsprechende Markhöhle eingebracht und bis zu den jeweiligen Markraumenden vorgeschoben, wobei sich die mit Spitzen versehenen Endkappen (Figur 1 Nr. 1) mit etwas Druck im knöchernen Markraumende rotationsstabil verankern können. Im nächsten Schritt werden die beiden Marknagelanteile ineinandergeschoben, dabei wird das Kreuzprofil des einen in ein entsprechendes Gegenstück (negativ eines Kreuzprofils Figur 1 Nr. 5) eingebracht. Die beiden Marknagelenden sind somit ebenfalls rotationsstabil verbunden und werden zusätzlich durch zwei Vollgewindeschrauben (Figur 1 Nr. 7), welche in zwei dafür vorgesehene gewindetragende Bohrungen (Figur 1 Nr. 6), fixiert.

## Patentansprüche

1. Marknagel mit einer Längsachse für einen Röhrenknochen,
wobei der Marknagel einen zweiteiligen länglichen Grundkörper aufweist, welcher sich im Wesentlichen über die Länge des Marknagels erstreckt, **dadurch gekennzeichnet,**
**dass** der Grundkörper mit einem sich entlang seiner Längsachse erstreckenden Kreuz- oder Sternprofil gebildet ist, welches im Wesentlichen sich entlang der Längsachse erstreckende Längskanten (3) aufweist, also der Grundkörper eine sich entlang seiner Längsrichtung erstreckende Außenkontur, mit einer Kreuz- oder Sternform im Querschnitt, aufweist, dessen Längskanten (3) sich parallel zur Längsrichtung des Grundkörpers erstrecken,
**dass** die beiden Teile des Grundkörpers an einander zugewandten Endseiten jeweils einen Teil eines Verschlussmechanismus zum Verbinden der Grundkörperteile miteinander aufweisen und
**dass** ein erster Teil des Verschlussmechanismus das Profil und/oder die Kontur des Grundkörpers, also die Kreuz- oder Sternform, aufweist und der zweite Teil des Verschlussmechanismus einen Aufnahmekörper (5) mit längs gerichteten Schlitzen aufweist, welche ihrer Form nach dem ersten Teil entsprechen.

2. Marknagel nach Anspruch 1
**dadurch gekennzeichnet,**
**dass** die Schlitze kreuz- oder sternförmig gebildet sind und/oder der Größe nach dem Profil und/oder der Kontur des ersten Teils entsprechen.

3. Marknagel nach Anspruch 1 oder 2
**dadurch gekennzeichnet,**
**dass** der Aufnahmeköper (5) in Längsrichtung zwischen dessen Schlitzen massiv ausgestaltet ist.

4. Marknagel nach einem der Ansprüche 1 bis 3
**dadurch gekennzeichnet,**
**dass** der Aufnahmekörper (5) zwischen den Schlitzen aus einzelnen Aufnahmekörperteilen gebildet ist, welche in ihrer Anordnung zueinander die Schlitze bilden und/oder dass die Aufnahmekörperteile auf und/oder zwischen einzelnen Segmenten der Kreuz- oder Sternform des Grundkörperteils angeordnet sind.

5. Marknagel nach einem der Ansprüche 1 bis 4
**dadurch gekennzeichnet,**
**dass** der Aufnahmekörper (5) Bohrungen (6) aufweist, über welche die ineinandergesteckten Teile des Verschlussmechanismus verschraubbar oder verbolzbar, also festlegbar, sind.

6. Marknagel nach einem der Ansprüche 1 bis 5
**dadurch gekennzeichnet,**
**dass** der Teil des Grundkörpers, welches den ersten Teil des Verschlussmechanismus trägt, hinter diesem ein Anschlag für den Aufnahmekörper (5) des zweiten Teils des Verschlussmechanismus aufweist, welcher vorzugsweise der Beschaffenheit, insbesondere der Außenkontur, des Aufnahmekörpers (5) entspricht.

7. Marknagel nach Anspruch 6,
**dadurch gekennzeichnet,**
**dass** der Anschlag fest mit dem ersten Teil des Grundkörpers und/oder der Aufnahmekörper (5) fest mit dem zweiten Teil des Grundkörpers verbunden ist.

8. Marknagel nach einem der Ansprüche 1 bis 7
**dadurch gekennzeichnet,**
**dass** der Aufnahmekörper (5) im Innern der Schlitze einen Anschlag für den ersten Teil des Verschlussmechanismus aufweist, welcher eine Eindringtiefe in den Aufnahmekörper begrenzt.
